# EUROPEAN PATENT APPLICATION

(11) **EP 2 028 466 A1**
(43) Date of publication of application: **25.02.2009**
(21) Application number: 06725828.5
(22) Date of filing: 22.03.2006
(51) Int. Cl.: G01L 1/14, G08B 13/10, G08B 13/26

(54) **CARPET WITH PRESENCE DETECTOR**

(71) Applicant: Asoc. de Investigación de la Industria Textil, 03800 Alcoy. (ES); Fundació Privada Universitat I Tecnología, 03330 Crevillente (ES); Unión Nacional de Fabricantes de Alfombras, 08022 Barcelona (ES)
(72) Inventor: CAMBRA SÁNCHEZ, Vicente, 46870 Ontinyent (Valencia) (ES); GISBERT GOMIS, José, 03801 Alcoy (Alicante) (ES); ESCUDERO COSTA, Francesc, 08784 Piera (Barcelona) (ES); LUENGO ALVAREZ, Sonia, 08028 Barcelona (ES)
(74) Representative: Ungria Lopez, Javier
(86) International application number: PCT/ES2006/000141
(87) International publication number: WO 2007/125133

(57) **Abstract**

The carpet includes two conventional fabric layers, an upper layer (1) and another, lower layer (2), the first being a visible decorative layer and the second being the base, and between the two layers (1) and (2), there is a structure forming a capacitor and formed by an insulating or dielectric layer (3) impregnated, on both surfaces, with conducting latex layers (4), this intermediate structure forming a capacitor whose capacitance variation derives from the difference in distance between the conducting layers (4) when a pressure is exerted on the actual carpet, either by a person or by an object. The carpet is supplemented with an electronic system capable of converting this distance variation into a signal, which, duly transformed, indicates the presence of an object or of a person located on the actual carpet.

## Description

### OBJECT OF THE INVENTION

As stated in the title of this specification, the present invention refers to a carpet with presence detector, In which a system is defined composed of different structures arranged in such a way that it is possible to detect the presence of an object or person located on that carpet, based on the measurement of the variation in electrical capacitance to which an electronic capacitor system is subjected to via the elements that form the structure of the carpet and of its own presence detector unit.

The object of the invention is to provide a carpet with a conventional aesthetic structure and appearance, which performs its function normally, but which incorporates means for permitting a presence on that carpet to be detected.

### PRIOR ART OF THE INVENTION

Carpets are currently created in very different ways, some with one or another type of fabric, with one or another type of weaving to produce the carpet, with one or another kind of dye and therefore of decorative motifs in order to provide the carpet with one or another ornamental or decorative appearance, etc. In other words, many different types of carpet are known, though they are always created and structured with a dual purpose of being decorative or ornamental and of protection for the floor, along with comfort for the user.

Nevertheless, the inventors are unaware of the existence of carpets with means that permit the presence of people or objects of them to be detected.

Presence detectors are of course known in certain sectors or fields of industry, but not applied to carpets.

### DESCRIPTION OF THE INVENTION

The inventive carpet is characterised by incorporating a presence detector system for people or objects located on it, the system being conceived with the capacity to act in the event of specific external stimuli, permitting the functional characteristic of the carpet to become an active function, one that is interactive with that of its environment and with wide-ranging new possibilities without ceasing to offer its other aesthetic functions and functions of use.

The detector system is based on the variation of a signal emitted by certain means which act as an electronic capacitor via the elements forming the structure of the carpet in the strict sense.

In that regard, the means which act as a capacitor are formed from two layers of conducting latex, arranged in parallel and physically separated by a dielectric with certain pre-established properties of deformation, recovery and useful life, all this in such a way that the variation in capacitance that is originated is proportional to the distance existing between the two conducting layers, thereby establishing the capacitance effect, with which the variation in distance will imply a variation In capacitance, thus permitting the detection of the corresponding change.

Structurally, the carpet comprises an upper layer of conventional fabric, which is the one that forms the outer, aesthetic and visible part; a lower layer functioning as a base, also conventional and made of fabric, and between the two layers is the structure corresponding to the detector system, wherein the capacitor is formed from the aforesaid conducting layers and the insulating or dielectric layer; while the electronic part is formed starting from two conducting wires connected to the two conducting layers, said two conductors being connected moreover to an electronic device which detects the variation in the signal produced by the capacitor system, once the voltage has been applied.

The conducting layers of the capacitor are preferably created from viscous latex mixed with short carbon fibre of length between 2 and 3 cm, the impregnation of the insulator is produced in a foulard where the upper and lower part of it is soaked in the latex thereby forming the two flat, parallel conducting layers with a completely smooth and continuous surface.

The upper and lower part of the carpet are also made to pass through the foulard prior to the incorporation of the electronic system, and the entire laminar structure is then passed through a rame which operates at between 100° and 150°C, depending on the kind of latex used, in order to carry out the final fixing and adhesion of the entire structure, resulting in a sandwich structure which maintains its form as a textile article without any variation at all in the aesthetic and use properties of the carpet.

In terms of the electronic system, this will preferably be materialised by an electronic plate designed and developed on a made-to-measure basis, which displays the capacitance when it detects the variation in the signal produced by the deformation of the capacitor assembly, once the voltage has been applied.

Said electronic system has a dual function, since on the one hand and in one direction, the capacitor system of the carpet is fed via the conducting wires, with a small voltage of around 12 volts provided by an external power supply source, while on the other hand and in the other direction it receives the variation in capacitance of that system, which, if different from the capacitance without deformation In a defined range of sensitivity, detects this and transforms it into a potential-free continuous electric signal.

### BRIEF ASCRIPTION OF THE DRAWINGS

In order to complement the description that is going to be made forthwith and with the aim of aiding a better understanding of the characteristics of the invention, this descriptive specification is accompanied by a set of drawings on the basis of which the innovations and advantages of the inventive carpet will be more easily comprehended.
Figure 1.- Shows a representation according to an exploded perspective of the different parts or elements forming the structure of the carpet that is the object of the invention, with the electronic system connected to it.
Figure 2.- Shows a view corresponding to a detail in cross-section of the carpet in the rest position.
Figure 3.- Shows a view of the same detail as in the above figure, with the carpet deformed as a consequence of pressure being exerted on it.

### DESCRIPTION OF AN EXAMPLE OF PREFERRED EMBODIMENT

With the commented figures in view, it can be seen how the inventive carpet is formed from a clearly differentiated dual structure, with one structure corresponding to the actual carpet, with the incorporation of a capacitor system as will be explained further below, while the other structure corresponds to an electronic system for detection which will also be described further below.

In relation to the structure of the carpet, this comprises an upper layer 1 of conventional manufacture, In other words, made of an appropriate fabric, and forming the external, visible and aesthetic part of the carpet, and a lower part 2 which forms the base of the actual carpet, also materialised in fabric, as is conventional in any carpet.

Those two upper 1 and lower 2 layers or parts of the carpet are complemented with an intermediate structure forming the capacitor system, named thus here because it acts as an electrical capacitor, being formed from an insulating or dielectric layer 3 with thickness, composition and useful life that can be variable, though it has to possess certain inherent properties of deformation and recovery that are adequate for guaranteeing that it is sensitive for the electronic detection system that is going to be described later on.

Applied to the upper and lower surface of said insulating or dielectric layer 3 are two conducting layers of latex, created by the impregnation of viscous latex mixed with short carbon fibres of length from 2 to 3 cm, those two conducting layers 4 and the intermediate dielectric layer 3 forming a single body or structure which is fixed between the two upper 1 and lower 2 layers of the actual carpet.

The capacitor system that forms the structure of the layers 3 and 4 acts overall like a capacitor system at the moment In which each of the latex layers 4 receives a certain voltage.

As far as the electronic system is concerned, it is created starting from two conductors 5 connected via one of their ends to the conducting layers 4, while the other end is connected to an electronic device 6 designed and developed on a made-to-measure basis, which displays the capacitance when it detects the variation In the signal produced by the deformation of the capacitor assembly, once the voltage has been applied.

Said electronic system 6 has a dual function, since on the one hand and in one direction, it is fed via the conducting wires 5 of the capacitor system formed by the layers 3 and 4, with a small voltage of around 12 volts provided by an external power supply source 7, while on the other hand and in the other direction the said electronic device 6 receives the variation in capacitance of the capacitor system, which, if different from the capacitance thereof without deformation in a defined range of sensitivity, detects this and transforms it into a potential-free continuous electric signal, via the appropriate electronic block or circuit 8.

As is evident, in the rest position of the carpet as represented in figure 1, the conducting latex layers 4 are separated by a distance "D" represented in that figure 1, while if a pressure is exerted on the upper part of the carpet, whether due to being trodden on by a person, or due to an object being deposited on it, the structure of the capacitor becomes deformed, of course, with the outer and upper layer 1 of the carpet, as represented in figure 2, where in this case the distance of the conducting layers 4 is "D1" represented in that figure 2. in such a way that when the distance is varied the capacitance also varies, so that on the basis of that variation in distance the external electronic system acts, detecting the change.

## Claims

1. **CARPET WITH PRESENCE DETECTOR,** structured by means of a conventional lower fabric layer (2) as the base and a conventional upper fabric layer (1), as the visible and decorative part of the carpet, is **characterised in that** it comprises an intermediate structure between said two layers, upper (1) and lower (2), with said intermediate structure comprising two separate and parallel conducting layers (4), with an insulating layer (3) of dielectric material between them, producing a capacitor that is deformable by pressure acting on it, capable of providing a variation in the electrical capacitance in its connection with an electronic detection system comprising two conductors (5) connected at one end, to the two conducting layers (4) and at the other, to an electronic device (6), capable of detecting the variation in signal caused by the deformation of the capacitor structure when the power supply is applied, with the electronic system being complemented by a external power supply (7) and with a circuit for transforming the detection into a potential-free continuous electric signal (8).

2. **CARPET WITH PRESENCE DETECTOR,** according to claim 1, **characterised in that** the two conducting layers (4) are preferably made of an impregnation of conducting latex that is applied over the upper and lower surfaces of the insulating or dielectric layer (3).

3. **CARPET WITH PRESENCE DETECTOR,** according to claim 2, **characterised in that** the latex conductor impregnation is viscous and mixed with short carbon fibres.

4. **CARPET WITH PRESENCE DETECTOR,** according to claims 2 and 3, **characterised in that** the two conducting layers (4) are flat, with smooth and continuous surfaces, and are separated by a predetermined distance (D1), which corresponds to the thickness of the insulating or dielectric layer (3).

5. **CARPET WITH PRESENCE DETECTOR,** according to claim 1, **characterised in that** the insulating or dielectric layer (3) presents pre-established characteristics of deformation, recovery and usage.

6. **CARPET WITH PRESENCE DETECTOR,** according to previous claims, **characterised in that** the electronic device (6) preferably comprises an electronic board with the capability of detecting the variation in the signal originated by the deformation of the capacitor array, once the voltage has been applied.

7. **CARPET WITH PRESENCE DETECTOR,** according to claim 6, **characterised In that** the electronic device (6), starting from the external power supply source (7), feeds the capacitor array, and is furthermore able to receive and detect the variation in capacitance for being transformed into a potential-free continuous electric signal by means of a circuit (8).
